# EUROPÄISCHE PATENTSCHRIFT

(11) **EP 3 536 348 B1**
(45) Veröffentlichungstag und Bekanntmachung des Hinweises auf die Patenterteilung: **29.10.2025**
(21) Anmeldenummer: 18160567.6
(22) Anmeldetag: 07.03.2018
(51) Int. Cl.: A61L 2/14, H05H 1/46

(54) **VERFAHREN ZUR BIOLOGISCHEN DEKONTAMINATION, DESINFEKTION ODER STERILISATION VON BEHANDLUNGSGUT MITTELS EINES REAKTIVGASES**
METHOD FOR BIO-DECONTAMINATION, DISINFECTION OR STERILIZATION OF OBJECTS WITH A REACTIVE GAS
PROCÉDÉ DE DÉCONTAMINATION BIOLOGIQUE, DE DÉSINFECTION OU DE STÉRILISATION DES PRODUITS À TRAITER AU MOYEN D'UN GAZ RÉACTIF

(43) Veröffentlichungstag der Anmeldung: 11.09.2019
(73) Patentinhaber: Leibniz-Institut für Plasmaforschung und Technologie e.V., 17489 Greifswald (DE)
(72) Erfinder: Ehlbeck, Jörg, 17498 Hinrichshagen (DE); Andrasch, Mathias, 18439 Stralsund (DE); Stachowiak, Jörg, 17489 Greifswald (DE); Schnabel, Uta, 17491 Greifswald (DE); Krohmann, Udo, 17033 Neubrandenburg (DE); Bösel, André, 17489 Greifswald (DE)
(74) Vertreter: Schulz Junghans Patentanwälte PartGmbB

(56) Entgegenhaltungen:
- EP-A1- 2 211 916
- WO-A1-2011/147595
- WO-A1-2012/112042
- WO-A2-2007/028813
- CA-A1- 2 224 118
- DE-A1- 102011 003 782
- US-A1- 2008 237 484
- PIPA A V ET AL: "Paper;Observation of microwave volume plasma ignition in ambient air;Observation of microwave volume plasma ignition in ambient air", PLASMA SOURCES SCIENCE AND TECHNOLOGY, INSTITUTE OF PHYSICS PUBLISHING, BRISTOL, GB, vol. 21, no. 3, 24 May 2012 (2012-05-24), pages 35009, XP020224247, ISSN: 0963-0252, DOI: 10.1088/0963-0252/21/3/035009

## Beschreibung

Die Erfindung betrifft ein Verfahren zur biologischen Dekontamination, Desinfektion oder Sterilisation von Behandlungsgut mittels eines Reaktivgases.

Biologische Dekontaminationsverfahren, insbesondere Desinfektions- und Sterilisationsverfahren sind Verfahren mit dem Ziel einer möglichst vollständigen Inaktivierung oder Abtötung von Mikroorganismen, wie z.B. Bakterien und hitzeresistenten Sporen. Diese Verfahren sind insbesondere in der Medizintechnik und in der Pharma- und Lebensmittelindustrie von großer Bedeutung. Für medizinische Anwendungen müssen beispielsweise Operationsbestecke zur Vermeidung von Infektionen keimfrei gemacht werden. Auch in der Lebensmittelindustrie sind Dekontaminationsverfahren essentiell, um eine Kontamination von Lebensmitteln mit gesundheitsschädlichen Mikroorganismen zu verhindern.

Aus dem Stand der Technik sind diverse Sterilisationsverfahren bekannt. Beispielsweise wird das zu sterilisierende Sterilisationsgut beim Autoklavieren bei erhöhtem Druck auf eine Temperatur von 121 °C gebracht. Zur Sterilisation von Festkörpern eignet sich auch eine trockene Heißluftsterilisation, beispielsweise bei 180 °C. Neben den genannten Hitzesterilisationsverfahren findet auch die Sterilisation durch ionisierende Strahlung, beispielsweise UV-Strahlung oder Gammastrahlung, breite Anwendung. Besonders für die Sterilisation von Festkörpern wird auch die chemische Sterilisation, z.B. durch Begasen mit Formaldehyd oder Ethylenoxid, eingesetzt.

Diesen Verfahren des Standes der Technik ist gemeinsam, dass sie entweder teure Apparaturen und hohe Energiekosten oder den Einsatz teurer und giftiger Chemikalien erfordern.

Aus dem Stand der Technik ist weiterhin ein Sterilisationsverfahren mittels plasmageneriertem Reaktivgas und eine Vorrichtung zur Generierung eines solchen Reaktivgases bekannt (WO2011138463A1, [0018], [0030]), mit dem sich insbesondere komplexe dreidimensionale Objekte antimikrobiell behandeln lassen. Dabei wird das Sterilisationsgut direkt einem Gasstrom des erzeugten Reaktivgases ausgesetzt. Ein Vorteil dieses Verfahrens ist die Möglichkeit, ohne teure und giftige Chemikalien ein Reaktivgas aus der Umgebungsluft als Prozessgas mittels eines Plasmas zu erzeugen. Das Verfahren hat allerdings den Nachteil, dass eine teure Plasmaquelle benötigt wird.

In der WO 2011/147595 A1 wird ein Sterilisationsverfahren offenbart, bei dem ein Gegenstand in einer Kammer einem Reaktivgas, das mit einem Plasma erzeugt wurde, ausgesetzt wird.

Die WO 2007/028813 A2 offenbart ein Verfahren zur Erzeugung eines Mikrowellenplasmas zur Behandlung von Oberflächen.

Pipa et al "Observation of Microwave Volume Plasma Ignition in Ambient Air", in Plasma Sources Science and Technology Vol. 21, (2012) 035009 offenbart eine Vorrichtung zur mikrowellenbasierten Plasmaerzeugung unter hohen Drücken.

WO 2012/112042 A1 offenbart ein Verfahren, bei dem die Samen mittels eines Luftstroms in einer Behandlungskammer fluidisiert werden und bei dem ein Plasma in der Kammer gezündet wird, das die Samen umgibt.

Weiterhin ist in der CA 2,224,118 A1 eine Vorrichtung zur Sterilisation eines Behandlungsgutes mittels eines Plasma-Wasserdampf Verfahrens offenbart.

Die DE 102011003782 A1 zeigt eine Reinigungsvorrichtung für einen Gegenstand zur Plasmabehandlung und Beaufschlagung mit Reaktivgas.

In der WO 2009/060213 A1 wird ein mikrowellen-induzierter Plasmasterilisator offenbart. Schließlich offenbart die US 2008/0237484 A1 eine Plasmavorrichtung zur Desinfektion von Wunden, die eine Kammer zur Ionisierung von Gas umfasst, das zur Desinfektion herangezogen wird. Infolgedessen ergibt sich die Aufgabe, ein einfaches, kostengünstiges und mobiles Sterilisationsverfahren unter Verwendung eines plasmagenerierten Reaktivgases zur Verfügung zu stellen.

Diese Aufgabe wird durch den Gegenstand des unabhängigen Anspruchs 1 gelöst.

Ausgestaltungen der Erfindung werden in den Unteransprüchen beansprucht und nachfolgend beschrieben.

Gemäß einem ersten Aspekt der Erfindung wird ein Verfahren zur Sterilisation von Sterilisationsgut mittels eines Reaktivgases zur Verfügung gestellt. Das Verfahren umfasst das Erzeugen eines Reaktivgases aus einem Prozessgas (Umgebungsluft) durch Zünden eines Plasmas, wobei das Reaktivgas (R) Stickstoffdioxid (NO₂) aufweist, und wobei das Reaktivgas (R) bei gleichem Druck und gleicher Temperatur eine höhere Dichte als Luft unter denselben Umgebungsbedingungen aufweist. Umgebungsbedingungen sind hierbei insbesondere die Temperatur und der Druck.

Die Dichte des Reaktivgases ist insbesondere mindestens in einem Temperaturbereich zwischen 30°C und 80°C und mindestens in einem Druckbereich zwischen 500 mbar und 2000 mbar größer als die von Luft unter den gleichen Bedingungen.

Insbesondere gilt daher, dass das Reaktivgas eine Dichte von mehr als 0,00129 g/cm³, insbesondere mehr als 0,003 g/cm³ , insbesondere 0,0036 g/cm³, bei 0 °C und Atmosphärendruck (1013 mbar) aufweist. Insbesondere ist die Dichte des Prozessgases mehr als doppelt so hoch wie die Dichte die von Luft unter den gleichen Bedingungen.

Das Verfahren umfasst weiterhin das Befüllen eines Behälters mit dem Reaktivgas, so dass der Behälter nahezu vollständig oder zumindest teilweise mit dem Reaktivgas gefüllt ist und die Sterilisation des Sterilisationsguts in dem Behälter mittels des Reaktivgases erfolgt.

Dabei beschreibt der Begriff "Reaktivgas" ein Gas, Gasgemisch oder eine Mischung eines oder mehrerer Gase und einer oder mehrerer Flüssigkeiten, welches reaktive Bestandteile mit einem Mindestvolumenanteil von 1 ppm (parts per million) aufweist, welche nach der Erzeugung an weiteren Reaktionen mit sich selbst oder anderen Bestandteilen des Reaktivgases teilnehmen, wobei die reaktiven Bestandteile insbesondere Radikale sein können. Diese chemischen Reaktionen bewirken einen Abbau der reaktiven Bestandteile, der bei den genannten Druck- und Temperaturverhältnissen so abläuft, dass die inaktivierende Wirkung des Reaktivgases ohne Nachproduktion des Reaktivgases und ohne Abbauprozess an in Kontakt stehenden Oberflächen zwischen 30 und 60 Minuten erhalten bleibt.

Das Reaktivgas weist eine höher Dichte als Luft auf, also insbesondere mehr als 0,00129 g/cm³ bei einer Temperatur von 0 °C und bei Atmosphärendruck, also einem Druck von etwa 1 bar, auf. Das bedeutet, dass das Reaktivgas bei dem angegebenen Druck und der angegebenen Temperatur eine größere Dichte aufweist als Umgebungsluft bei dem gleichen Druck und der gleichen Temperatur. Somit verdrängt das Reaktivgas insbesondere in einem nach oben offenen Behälter die Umgebungsluft und sackt in dem Behälter nach unten.

Infolgedessen lässt sich das Reaktivgas in einem nach oben offenen Behälter über einen gewissen Zeitraum lagern. Zusätzlich lässt sich das Reaktivgas aufgrund seiner höheren Dichte im Vergleich zur Umgebungsluft unter Ausnutzung der Schwerkraft in einen Behälter einfüllen. Diese Eigenschaften ermöglichen insbesondere die Ausführung des erfindungsgemäßen Dekontaminationsverfahrens als Tauchverfahren, wobei Behandlungsgut in einen mit Reaktivgas gefüllten Behälter eingetaucht wird. Hierdurch wird der mechanische Aufwand für das Verfahren durch das Entfallen von Türen und Dichtungen entscheidend reduziert und Reaktivgasverluste durch das Ein- und Ausschleusen des Behandlungsguts reduziert. Dadurch werden Verfahrenskosten reduziert und die Durchsatzleistung erhöht.

Weiterhin ermöglicht die höhere Dichte des Reaktivgases im Vergleich zur Umgebungsluft das Abfüllen von Reaktivgas in Behälter oder Verpackungen zur späteren Dekontamination des Behandlungsgutes in diesen Behältern oder Verpackungen. In diesem Fall kann der Dekontaminationsvorgang auch während des Transports der Behälter oder Verpackungen erfolgen, ohne dass sich der Behälter oder die Verpackung am Ort der Plasmaquelle befinden muss.

Gemäß der Erfindung wird als Prozessgas Umgebungsluft verwendet. Gemäß der Erfindung weist das Reaktivgas Stickstoffdioxid (NO₂) auf.

Insbesondere ist das zum Erzeugen des Reaktivgases aus dem Prozessgas verwendete Plasma ein Niedertemperatur-Atmosphärendruckplasma.

Solche Plasmen können insbesondere mittels Bogenentladungen, Corona-Entladungen, dielektrisch behinderten Entladungen (DBD), Atmosphärendruck-Plasmajets (APPJ), Hochfrequenzentladungen, wie z.B. inductive coupled plasma (ICP) oder mikrowellengetriebenen Entladungen wie z.B. microwave torches erzeugt werden.

Der Begriff "Behandlungsgut" bezeichnet mindestens einen zu dekontaminierenden Festkörper oder mindestens eine zu dekontaminierende Flüssigkeit. Insbesondere kann auch der mit Reaktivgas gefüllte Behälter selbst das Behandlungsgut darstellen.

Gemäß der Erfindung wird das Behandlungsgut in den mit Reaktivgas gefüllten Behälter abgesenkt, so dass das Sterilisationsgut vollständig von dem Reaktivgas bedeckt ist, wobei nach Ablauf einer Einwirkzeit das Behandlungsgut dem Behälter entnommen wird.

Solche Verfahren werden auch als Tauchverfahren bezeichnet. Bei entsprechenden Verfahren können auf einfache Weise eine große Anzahl von Behandlungsgütern nacheinander, insbesondere automatisiert dekontaminiert werden. Dabei muss nicht vor jedem Behandlungsvorgang neues Reaktivgas in den Behälter gefüllt werden, sondern in dem Behälter befindliches Reaktivgas kann auch für mehrere Dekontaminationsvorgänge mehrerer Behandlungsgüter verwendet werden, sofern die Konzentration reaktiver Bestandteile in dem Reaktivgas noch ausreichend hoch ist, um eine Dekontamination zu ermöglichen. Ein entsprechendes Tauchverfahren kann auch räumlich getrennt von der Plasmaquelle ausgeführt werden.

Gemäß einer weiteren Ausführungsform wird das Einbringen des Behandlungsguts in den Behälter und/ oder das Entnehmen des Behandlungsguts aus dem Behälter mittels einer Hebe- und/ oder Senkvorrichtung, insbesondere automatisiert, ausgeführt.

Gemäß einer weiteren Ausführungsform wird das Transportieren des Behandlungsguts zu dem Behälter und/ oder von dem Behälter weg mittels einer Fördervorrichtung, beispielsweise eines Förderbandes, insbesondere automatisiert, ausgeführt.

Gemäß einer weiteren Ausführungsform des Verfahrens ist der Behälter während des Befüllens des Behälters nach oben zumindest teilweise offen oder geöffnet. Hierdurch erübrigt sich ein Öffnen des Behälters vor dem Einbringen des Behandlungsguts.

Gemäß einer weiteren Ausführungsform (nicht Teil der Erfindung wie in den Ansprüchen ausgeführt) des Verfahrens wird vor dem Einleiten des Reaktivgases in den Behälter das Behandlungsgut in dem Behälter platziert, so dass das Behandlungsgut mittels des in den Behälter eingeleiteten Reaktivgases dekontaminiert wird. Gemäß einer weiteren Ausführungsform des Verfahrens wird das Behandlungsgut während des Transports des Behälters dekontaminiert. Bei dieser Ausführungsform kann Zeit für den Dekontaminationsvorgang eingespart werden.

Der Absorber ist gemäß dieser Ausführungsform insbesondere so konfiguriert oder dimensioniert, dass die Konzentration des Reaktivgases spätestens bis zum Zeitpunkt des Öffnens der Verpackung unterhalb eines die Gesundheit gefährdendes Maß abgesunken ist. Gemäß der Erfindung wird das Behandlungsgut nach der Dekontamination aus dem Behälter entfernt.

Gemäß einer weiteren Ausführungsform des Verfahrens wird ein erstes Behandlungsgut derart in dem Behälter platziert, dass das erste Behandlungsgut von dem in dem Behälter befindlichen Reaktivgas bedeckt ist, so dass das erste Behandlungsgut mittels des in dem Behälter befindlichen Reaktivgases dekontaminiert wird, und wobei nach der Dekontamination des ersten Behandlungsguts das erste Behandlungsgut aus dem Behälter entfernt wird, und wobei ein zweites Behandlungsgut nach dem Entfernen des ersten Behandlungsguts aus dem Behälter derart in dem Behälter platziert wird, dass das zweite Behandlungsgut von dem in dem Behälter befindlichen Reaktivgas bedeckt ist, so dass das zweite Behandlungsgut mittels des in dem Behälter befindlichen Reaktivgases dekontaminiert wird.

Gemäß einer weiteren Ausführungsform des Verfahrens wird der Behälter nach dem Befüllen des Behälters mit dem Reaktivgas verschlossen, insbesondere luftdicht versiegelt. Durch ein Verschließen des Behälters verbleibt das Reaktivgas, insbesondere während des Transports, länger in dem Behälter. Außerdem wird durch das Verschließen ein erneutes Einbringen von Keimen in den Behälter verhindert.

Gemäß einer weiteren Ausführungsform des Verfahrens ist der Behälter eine Verpackung, insbesondere eine Tiefziehverpackung oder eine Beutelverpackung, wobei die Verpackung nahezu vollständig mit dem Reaktivgas befüllt wird, und wobei die Verpackung nach dem Befüllen mit dem Reaktivgas verschlossen wird.

Dabei kann Reaktivgas in die Verpackung eingebracht werden, während sich bereits Sterilisationsgut in der Verpackung befindet (nicht Teil der Erfindung wie in den Ansprüchen ausgeführt), oder das Sterilisationsgut kann nachträglich in die Verpackung eingebracht werden, wobei das Reaktivgas zumindest teilweise verdrängt wird.

Insbesondere wird die Verpackung nach Befüllen mit dem Reaktivgas verschlossen, insbesondere gasdicht versiegelt. Durch ein Verschließen der Verpackung verbleibt das Reaktivgas, insbesondere während des Transports länger in der Verpackung. Außerdem wird durch das Verschließen ein erneutes Einbringen von Mikroorganismen in die Verpackung verhindert.

Gemäß einer weiteren Ausführungsform besteht die Verpackung zumindest teilweise aus Pappe oder Papier. Pappe oder Papier wirkt in Bezug auf das Reaktivgas als Adsorber und nimmt reaktive Bestandteile auf bzw. baut reaktive Bestandteile ab. Auf diese Weise lässt sich die Einwirkzeit des Reaktivgases auf das in dem Behälter befindliche Behandlungsgut vermindern bzw. den Prozessanforderungen anpassen. Mittels einer Beschichtung oder Laminierung der Pappe oder des Papiers oder durch Bereitstellen von Einlegefolien lässt sich die Adsorber- bzw. Abbauwirkung der Pappe oder des Papiers vermindern, was zu einer längeren Einwirkzeit des Reaktivgases führt.

Gemäß einer weiteren Ausführungsform des Verfahrens ist der Behälter ein Flüssigkeitsbehälter, insbesondere eine Flasche, wobei der Flüssigkeitsbehälter nahezu vollständig mit dem Reaktivgas befüllt wird, und wobei der Flüssigkeitsbehälter nach dem Befüllen mit dem Reaktivgas verschlossen wird.

In diesem Fall ist das Sterilisationsgut insbesondere eine Flüssigkeit, wobei eine Sterilisation der Flüssigkeit durch Durchmischen der Flüssigkeit mit dem Reaktivgas erfolgen kann. Die Flüssigkeit kann sich bereits zum Zeitpunkt des Befüllens des Flüssigkeitsbehälters mit dem Reaktivgas in dem Flüssigkeitsbehälter befinden (nicht Teil der Erfindung wie in den Ansprüchen ausgeführt). Alternativ dazu kann die Flüssigkeit nach dem Befüllen des Flüssigkeitsbehälters mit dem Reaktivgas in den Flüssigkeitsbehälter eingebracht werden, wobei das Reaktivgas zumindest teilweise verdrängt wird.

Durch ein Verschließen des Flüssigkeitsbehälters verbleibt das Reaktivgas, insbesondere während des Transports, länger in der Verpackung. Außerdem wird durch das Verschließen ein erneutes Einbringen von Mikroorganismen in den Flüssigkeitsbehälter verhindert. Gemäß einer weiteren Ausführungsform des Verfahrens wird mittels eines Adsorbers/Abbauers das in dem Behälter befindliche Reaktivgas aufgenommen oder abgebaut. Alternativ zu den oben beschriebenen Pappe oder Papier aufweisenden Verpackungen können externe Adsorber/Abbauer, beispielsweise aus Zellstoff oder Aktivkohle, in den Behälter eingebracht oder dessen Oberflächen mit entsprechend wirkenden Materialien beschichtet oder getränkt werden, um die Einwirkzeit des Reaktivgases auf das Behandlungsgut zu vermindern.

Gemäß einer weiteren Ausführungsform des Verfahrens umfasst das Prozessgas Wasserdampf.

Prozessgas, welches Wasserdampf aufweist, verbessert die Wirkung der Dekontamination.

Gemäß einer weiteren Ausführungsform des Verfahrens wird der Wassergehalt des Reaktivgases durch ein Wasserreservoir im Behälter oder in einem Reservoir im Behälter oder im Behälter selbst eingestellt. Dabei kann das Wasser auch in einem Absorbermaterial oder einem porösen Werkstoff gespeichert sein.

Gemäß der Erfindung wird das Behandlungsgut nach Ablauf einer Dekontaminationszeit zum Entfernen des Reaktivgases mit einem Spülgas, insbesondere mit steriler Luft, gespült. Hierbei können Reste des Reaktivgases von dem Behandlungsgut entfernt werden, insbesondere um die Konsumenten- bzw. Patientensicherheit zu gewährleisten.

Gemäß der Erfindung wird das Behandlungsgut an einer Spülposition mit Spülgas gespült, wobei insbesondere das Behandlungsgut automatisiert, beispielsweise mittels einer Fördervorrichtung zu der Spülposition transportiert wird.

Gemäß einer weiteren Ausführungsform des Verfahrens wird das Reaktivgas nach Abschluss des Dekontaminationsvorgangs, insbesondere durch Ausgießen, aus dem Behälter entfernt.

Gemäß einer weiteren Ausführungsform des Verfahrens wird das Plasma zur Erzeugung des Reaktivgases aus dem Prozessgas mittels Mikrowellen gezündet.

Geeignete mikrowellengetriebene Plasmaquellen weisen insbesondere einen Mikrowellengenerator, beispielsweise unter Verwendung eines Magnetrons, eine Mikrowellenzuführung, beispielsweise einen Hohlleiter oder ein Koaxialleitung, und einen mit einem Prozessgas gefüllten Entladungsraum auf, in dem Elektronen durch Resonanz mit den Mikrowellen kinetische Energie aufnehmen, was in der Bildung eines Plasmas resultiert. Grundsätzlich funktionieren entsprechende Plasmaquellen ohne das Vorhandensein von Elektroden. In manchen Konfigurationen weist der Entladungsraum eine zusätzliche Plasmazündeinrichtung auf, die das Zünden des Plasmas bei geringeren elektrischen Feldern ermöglicht. Die Plasmazündeinrichtung trägt dabei durch Resonanz mit den Mikrowellen und je nach Form der Plasmazündeinrichtung durch Spitzeneffekte zur Generierung des Plasmas bei.

Gemäß einer weiteren Ausführungsform dient als Plasmaquelle eine Haushaltsmikrowelle, insbesondere bei Verwendung einer erfindungsgemäßen Plasmazündeinrichtung. Dabei dient der Innenraum der Haushaltsmikrowelle bzw. die Umgebung der Plasmazündeinrichtung als Entladungsraum.

Gemäß einer weiteren Ausführungsform des Verfahrens weisen die Mikrowellen eine maximale Leistungsdichte von 0,05 bis 1 MW/m², insbesondere 0,2 bis 0,5 MW/m², auf.

Entsprechende Energiedichten können mittels kostengünstiger handelsüblicher Haushaltsmikrowellenöfen erzeugt werden, wodurch sich bei Vorhandensein einer geeigneten Plasmazündeinrichtung eine teure externe Plasmaquelle erübrigt.

Gemäß einer weiteren Ausführungsform des Verfahrens erfolgt der

Dekontaminationsvorgang während der Generierung des Plasmas mittels der Mikrowellen. Alternativ dazu kann zunächst mittels der Mikrowellen ein Reaktivgas in dem Behälter erzeugt werden und anschließend das Behandlungsgut in den Behälter eingebracht werden. Dies ist insbesondere bei der Dekontamination von metallhaltigen Festkörpern sinnvoll, die nicht der Mikrowellenstrahlung ausgesetzt werden sollen.

Gemäß einer weiteren Ausführungsform des Verfahrens umfasst das Prozessgas bei der Generierung des Reaktivgases aus dem Prozessgas durch ein mikrowellengetriebenes Plasma Wasserdampf.

Gemäß einer Ausführungsform des Verfahrens wird bei der Generierung des Reaktivgases aus dem Prozessgas durch ein Plasma, insbesondere durch ein mikrowellengetriebenes Plasma oder nach der Generierung des Reaktivgases durch ein Plasma, insbesondere durch ein mikrowellengetriebenes Plasma, das Reaktivgas mittels eines Adsorbers aufgenommen oder abgebaut.

Gemäß einer weiteren Ausführungsform des Verfahrens wird das Reaktivgas nach der Generierung des Reaktivgases durch ein Plasma, insbesondere durch ein mikrowellengetriebenes Plasma nach Abschluss des Sterilisationsvorgangs, insbesondere durch Ausgießen, aus dem Behälter entfernt.

Gemäß einer weiteren Ausführungsform des Verfahrens wird das Reaktivgas nach der Generierung des Reaktivgases durch ein Plasma, insbesondere durch ein mikrowellengetriebenes Plasma nach Abschluss des Dekontaminationsvorgangs, durch Spülen mit Frischgas/Sterilluft entfernt.

Gemäß einer weiteren Ausführungsform des Verfahrens wird das Plasma mittels einer Plasmazündeinrichtung nach dem zweiten Aspekt der Erfindung gezündet.

Ein zweiter Aspekt der Offenbarung, der nicht von den Ansprüchen umfasst ist, betrifft eine Plasmazündeinrichtung, umfassend eine elektrische leitfähige Grundplatte und mindestens zwei mit der Grundplatte elektrisch leitend verbundene elektrisch leitfähige Stifte. Insbesondere umfassen die elektrisch leitfähigen Stifte Wolfram und sind z.B. aus Wolframdraht gefertigt. Dabei ist die Plasmazündeinrichtung dazu ausgebildet, durch Resonanz mit Mikrowellen einer maximalen Leistungsdichte von 0,05 bis 1 MW/m², insbesondere 0,2 bis 0,5 MW/m², zwischen den Stiften ein elektrisches Feld von typisch mehr als 0,1 kV/mm zu erzeugen, wobei mittels des elektrischen Feldes ein Plasma zündbar ist.

Zusätzlich zum Effekt der Resonanz mit den Mikrowellen können auch durch die Form der Plasmazündeinrichtung bedingte Spitzeneffekte zur Erzeugung des für die Zündung erforderlichen elektrischen Feldes beitragen.

Eine derartige Plasmazündeinrichtung ist mit sehr geringen Herstellungskosten und Fertigungsaufwand produzierbar.

Gemäß einer Ausführungsform ist die Plasmazündeinrichtung als Deckel für einen Behälter, insbesondere eine Flasche, ausgeführt. Bei einer solchen Ausführungsform kann mittels Mikrowellen in dem geschlossenen Behälter ein Plasma erzeugt werden, um ein Reaktivgas zur Dekontamination des Behandlungsguts bereitzustellen. Der Deckel kann in diesem Fall während des Transports auf dem Behälter verbleiben und eine Sterilisation während des Transports ermöglichen. Alternativ dazu können mittels einer als Decke ausgeführten Plasmazündeinrichtung passende Behälter selbst sterilisiert werden.

Gemäß einer weiteren Ausführungsform der Plasmazündeinrichtung weist mindestens ein Stift ein reaktives Abbrandmaterial oder ein antimikrobiell wirkendes Material, insbesondere Kohlenstoff, Kupfer oder Silber, auf. Dabei bezeichnet der Begriff "reaktives Abbrandmaterial" ein Material, das während einer Plasmaentladung von der Plasmazündeinrichtung durch Ätz- oder Sputterprozesse abgelöst wird und eine chemische Reaktion mit Bestandteilen des Plasmas eingehen kann und eine prozessfördernde Wirkung hat.

Gemäß einer weiteren Ausführungsform der Plasmazündeinrichtung ist mindestens ein Stift aus reaktivem Abbrandmaterial gefertigt oder mit reaktivem Abbrandmaterial oder einem antimikrobiell wirkendem Material beschichtet.

Gemäß einer weiteren Ausführungsform ist die Plasmazündeinrichtung in einem Haushaltsmikrowellenofen positionierbar.

Ein dritter Aspekt der Offenbarung, der nicht von den Ansprüchen umfasst ist, betrifft ein System zur Dekontamination von Behandlungsgut mittels eines Reaktivgases, insbesondere mit einem Verfahren nach dem ersten Aspekt der Erfindung, wobei das System zumindest die folgenden Komponenten aufweist: einen Behälter zur Aufnahme von Behandlungsgut und eine in dem Behälter positionierbare Plasmazündeinrichtung nach dem zweiten Aspekt der Erfindung. Dabei ist aus einem in dem Behälter befindlichen Prozessgas mittels des von der Plasmazündeinrichtung erzeugten Plasmas ein Reaktivgas erzeugbar, wenn die Plasmazündeinrichtung in dem Behälter angeordnet ist, so dass in dem Behälter befindliches Behandlungsgut mittels des erzeugten Reaktivgases sterilisierbar ist.

Dabei ist das System so dimensioniert, dass es in einer handelsüblichen Haushaltsmikrowelle positionierbar ist.

Bei Verwendung einer kostengünstigen Haushaltsmikrowelle in Kombination mit der kostengünstigen Plasmazündeinrichtung kann ein erfindungsgemäßes System zur Dekontamination mit extrem geringem Aufwand und zu extrem geringen Kosten zur Verfügung gestellt werden. Zudem ist ein solches System leicht transportabel und einfach und schnell aufzubauen. Diese Eigenschaften sind insbesondere vorteilhaft für einen Einsatz in Gebieten mit eingeschränkter Infrastruktur, z.B. beim Ausbruch von Seuchen in Ländern der Dritten Welt.

Gemäß einer Ausführungsform des Systems weist der Behälter einen Adsorber zur Aufnahme oder zum Abbau des Reaktivgases auf.

Gemäß einer weiteren Ausführungsform des Systems ist der Behälter nach oben zumindest teilweise offen oder geöffnet.

Gemäß einer weiteren Ausführungsform des Systems ist der Behälter ein Flüssigkeitsbehälter, insbesondere eine Flasche.

Gemäß einer weiteren Ausführungsform des Systems ist der Behälter eine Verpackung, insbesondere eine Tiefziehverpackung oder eine Beutelverpackung.

Gemäß einer weiteren Ausführungsform weist das System einen Flüssigkeitsbehälter zur Aufnahme einer Flüssigkeit auf.

Mittels einer in dem Flüssigkeitsbehälter befindlichen Flüssigkeit, die während der Erzeugung des Reaktivgases verdampft, kann eine Sterilisation mit höherer Effektivität durchgeführt werden.

Gemäß einem vierten Aspekt der Erfindung wird eine Vorrichtung zur Dekontamination von Behandlungsgut zur Verfügung gestellt.

Die Vorrichtung umfasst eine Plasmaquelle und einen Behälter zur Aufnahme von Behandlungsgut, wobei in dem Behälter mittels der Plasmaquelle ein Reaktivgas aus einem Prozessgas erzeugbar ist oder wobei mittels einer Reaktivgasleitung ein Reaktivgas in den Behälter einleitbar ist.

Die beschriebene Vorrichtung ist zur Ausführung des Verfahrens nach dem ersten Aspekt der Erfindung geeignet, wobei das Verfahren insbesondere als Tauchverfahren ausführbar ist.

Als Plasmaquellen können hierbei Vorrichtungen zur Erzeugung von Bogenentladungen, Corona-Entladungen, dielektrisch behinderten Entladungen (DBD), Atmosphärendruck-Plasmajets (APPJ), Hochfrequenzentladungen (z.B. ICP) oder mikrowellengetriebenen Entladungen, insbesondere Vorrichtungen zur Erzeugung von mikrowellengetriebenen Entladungen, eingesetzt werden, beispielsweise eine zu der in der Patentanmeldung WO2011138463A1 beschriebenen Vorrichtung analoge Vorrichtung.

Gemäß einer Ausführungsform weist die Vorrichtung ein Überlauf- und Spülbecken zur Aufnahme von aus dem Behälter austretendem Reaktivgas auf, wobei der Behälter in dem Überlauf- und Spülbecken angeordnet ist.

Gemäß einer weiteren Ausführungsform weist die Vorrichtung eine Abluftleitung zum Entfernen von aus dem Behälter ausgetretenen Reaktivgas aus dem Überlauf- und Spülbecken auf.

Ein derartiges Überlauf- und Spülbecken erhöht die Sicherheit bei der Benutzung durch Verhinderung des Austretens von Reaktivgas.

Gemäß einer weiteren Ausführungsform weist die Vorrichtung eine Spülposition zum Einleiten von Spülgas, insbesondere steriler Luft auf.

Mittels der Spülposition können Reste des Reaktivgases von dem Behandlungsgut entfernt werden.

Gemäß einer weiteren Ausführungsform weist die Vorrichtung eine Hebe- und Senkvorrichtung zum Absenken des Behandlungsgut in den Behälter und Entnehmen des Behandlungsguts aus dem Behälter auf. Insbesondere ist die Hebe- und Senkvorrichtung automatisiert betreibbar.

Gemäß einer weiteren Ausführungsform weist die Vorrichtung eine Transportvorrichtung zum Positionieren des Behandlungsguts über dem Behälter und/ oder über der Spülposition und/ oder zum Zuführen des Behandlungsguts zu der Vorrichtung und/ oder zum Abtransportieren des Behandlungsguts von der Vorrichtung auf. Insbesondere ist die Transportvorrichtung automatisiert betreibbar.

Alternativen einzelner trennbarer hier als Ausgestaltungen der Erfindung beschriebener Merkmale können frei kombiniert werden, um weitere Ausgestaltungen der Erfindung zu erhalten.

Weitere Merkmale und Vorteile der Erfindung werden nachfolgend durch Beschreibung von Ausführungsbeispielen anhand von Figuren erläutert.

Es zeigen
- Fig. 1: eine schematische Darstellung einer Vorrichtung zur Durchführung eines erfindungsgemäßen Tauchdekontaminationsverfahrens,
- Fig. 2: eine schematische Darstellung eines Systems zur Dekontamination von Behandlungsgut mittels eines Reaktivgases, und
- Fig. 3: eine schematische Darstellung zur Durchführung des Verfahrens mittels einer Förderstrecke.

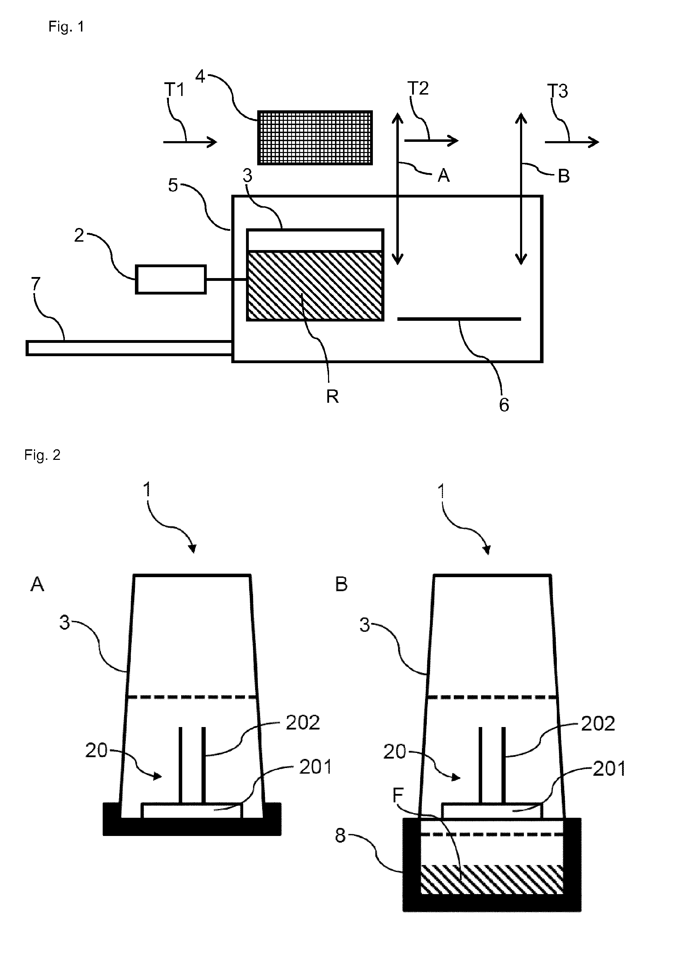

Im Einzelnen zeigt die Fig. 1 eine Schnittdarstellung einer Vorrichtung mit einem Überlauf- und Spülbecken 5, einem innerhalb des Überlauf- und Spülbeckens 5 angeordneten Behälter 3 zur Aufnahme von Behandlungsgut 4 und einer innerhalb des Überlauf- und Spülbeckens 5 angeordneten Spülposition 6. In dem Behälter 3 befindet sich ein mittels eines Plasmas erzeugtes Reaktivgas R. Der Behälter 3 ist weiterhin über eine Reaktivgasleitung mit einer Plasmaquelle 2 zur Erzeugung eines Plasmas verbunden. Über die Reaktivgasleitung kann mittels der Plasmaquelle 2 erzeugtes Reaktivgas R in den Behälter 3 eingeleitet werden.

Im oberen Teil der Fig. 1 ist der Ablauf eines beispielhaften erfindungsgemäßen Tauchdekontaminierungsverfahrens schematisch dargestellt. Das Behandlungsgut 4, beispielsweise ein zu dekontaminierenden Festkörper, wird in einem ersten Transportschritt T1 über dem Behälter 3 positioniert. In einem Dekontaminationsschritt A wird das Behandlungsgut 4 in den Behälter 3 abgesenkt, so dass das Behandlungsgut 4 vollständig von dem in dem Behälter 3 befindlichen Reaktivgas R bedeckt ist und für eine definierte Dekontaminationszeit in dem Behälter 3 belassen, so dass das Behandlungsgut 4 mittels des Reaktivgases R dekontaminiert wird und nach Ablauf der Dekontaminierungszeit dem Behälter 3 entnommen. In einem zweiten Transportschritt T2 wird das Behandlungsgut 4 über der Spülposition 6 positioniert. Anschließend wird das Behandlungsgut 4 in einem Spülschritt B auf die Spülposition 6 abgesenkt, mit einem Spülgas, insbesondere mit steriler Luft, gespült, wobei dem Behandlungsgut 4 anhaftende Reste von Reaktivgas R entfernt werden, und der Spülposition 6 entnommen. In einem dritten Transportschritt T3 wird das Behandlungsgut 4 für eventuelle weitere Schritte oder zur Entnahme weitertransportiert.

Die Transportschritte T1,T2,T3 können dabei beispielsweise mittels Förderbändern, insbesondere automatisiert, ausgeführt werden.

Das Absenken und Anheben innerhalb des Dekontaminierungsschritts A und/ oder des Spülschritts B kann beispielsweise mittels einer Hebe- und Senkvorrichtung, insbesondere automatisiert, ausgeführt werden.

An dem Überlauf- und Spülbecken 5 ist eine Abluftleitung 7 angeordnet, wobei mittels der Abluftleitung 7 aus dem Behälter 3 ausgetretenes Reaktivgas R aus dem Überlauf- und Spülbecken 5 entfernbar ist.

Die Fig. 2A zeigt eine Schnittdarstellung eines Systems 1 zur Dekontamination von Behandlungsgut mittels eines Reaktivgases. Das System 1 umfasst einen Behälter 3 zur Aufnahme von Behandlungsgut 4 und eine in dem Behälter 3 angeordnete Plasmazündeinrichtung 20 mit einer elektrisch leitfähigen Grundplatte 201 und zwei mit der Grundplatte 201 verbundenen elektrisch leitfähigen Stiften 202.

Die Fig. 2B zeigt eine Schnittdarstellung eines analog zu dem in Fig. 2A gezeigten System ausgeformten Systems 1 mit einem zusätzlichen Flüssigkeitsbehälter 8, der eine Flüssigkeit F enthält.

Die gezeigten Systeme 1 sind so dimensioniert, dass sie in einem handelsüblichen Haushaltsmikrowellenofen positionierbar sind.

Die gezeigte Plasmazündeinrichtung 20 ist derart ausgelegt, dass bei Einstrahlung von Mikrowellen einer maximalen Energiedichte von über 0,1 MW/m², was der Energiedichte eines handelsüblichen Haushaltsmikrowellenofens entspricht, durch Resonanz mit den Mikrowellen und gegebenenfalls Spitzenphänomene ein ausreichendes elektrisches Feld zwischen den Stiften 202 erzeugt wird, um ein Plasma mit Umgebungsluft als Prozessgas zu erzeugen. Somit kann aus einem Prozessgas mittels des Plasmas in dem Behälter 3 ein Reaktivgas R erzeugt werden, wenn die Plasmazündreinrichtung 20 geeigneter Mikrowellenstrahlung ausgesetzt ist. In dem Behälter 3 positioniertes Sterilisationsgut 4 kann somit mittels des Reaktivgases R dekontaminiert werden.

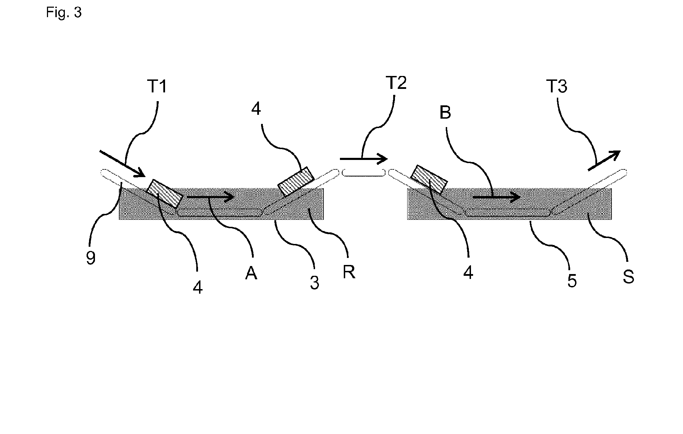

Die Fig. 3 zeigt eine weitere Variante des Verfahrens (nicht Teil der Erfindung wie in den Ansprüchen ausgeführt). Dabei wird das Behandlungsgut 4 zur Dekontamination auf einem Förderband 9 angeordnet. Das Förderband 9 transportiert (erster Transportschritt T1) das Behandlungsgut 4 in ein Behandlungsbecken 3, das mit dem Reaktivgas R befüllt ist, so dass das Behandlungsgut 4 im Reaktivgas R eingetaucht A wird. Nachdem das Behandlungsgut 4 im Behandlungsbecken 3 dekontaminiert, desinfiziert und/oder sterilisiert wurde, wird das Behandlungsgut 4 durch das Förderband 9 sodann in ein Spülbecken verbracht T2,B, wo das Reaktivgas R ausgespült wird, so dass potenziell im Behandlungsgut 4 verbliebenes gesundheitsschädliches Reaktivgas R entfernt wird. Das Spülbecken kann mit einer Flüssigkeit F oder einem Spülgas S befüllt sein. Anschließend wird das Behandlungsgut 3 mit dem Förderband 9 aus dem Spülbecken 5 heraus transportiert T3.

Auf diese Weise kann das Behandlungsgut seriell und vollautomatisch dekontaminiert werden.

### Bezugszeichenliste

- 1: System zur Dekontamination von Behandlungsgut
- 2: Plasmaquelle
- 20: Plasmazündeinrichtung
- 201: Grundplatte
- 202: Stift
- 3: Behälter
- 4: Behandlungsgut
- 5: Überlauf- und Spülbecken
- 6: Spülposition
- 7: Abluftleitung
- 8: Flüssigkeitsbehälter
- 9: Förderband
- R: Reaktivgas
- S: Spülgas
- F: Flüssigkeit
- A: Dekontaminationsschritt
- B: Spülschritt
- T1: Transportschritt 1
- T2: Transportschritt 2
- T3: Transportschritt 3

## Patentansprüche

1. Tauchdekontaminationsverfahren zur biologischen Dekontamination von Behandlungsgut (4) mittels eines Reaktivgases (R), umfassend die Schritte:
• Erzeugen eines Reaktivgases (R) aus einem Prozessgas durch Zünden eines Plasmas, wobei als Prozessgas Umgebungsluft verwendet wird und das Reaktivgas (R) Stickstoffdioxid (NO₂) aufweist, und wobei das Reaktivgas (R) bei gleichem Druck und gleicher Temperatur eine höhere Dichte als Luft aufweist,
• Befüllen eines Behälters (3) mit dem Reaktivgas (R), so dass der Behälter (3) nahezu vollständig oder zumindest teilweise mit dem Reaktivgas (R) gefüllt ist,
• Biologische Dekontamination des Behandlungsguts (4) in dem Behälter (3) mittels des Reaktivgases (R) wobei das Behandlungsgut (4) in den mit Reaktivgas (R) gefüllten Behälter (3) abgesenkt wird, so dass das Behandlungsgut vollständig von dem Reaktivgas bedeckt ist, wobei nach Ablauf einer Dekontaminationszeit das Behandlungsgut dem Behälter entnommen wird,
• wobei das Behandlungsgut nach Ablauf der Dekontaminationszeit zum Entfernen des Reaktivgases mit einem Spülgas an einer Spülposition gespült wird, um Reste des Reaktivgases von dem Behandlungsgut zu entfernen.

2. Verfahren nach Anspruch 1, wobei der Behälter (3) über eine Reaktivgasleitung mit einer Plasmaquelle (2) zur Erzeugung eines Plasmas verbunden ist und wobei über die Reaktivgasleitung mittels der Plasmaquelle (2) erzeugtes Reaktivgas (R) in den Behälter (3) eingeleitet wird.

3. Verfahren nach einem der vorhergehenden Ansprüche, wobei das Behandlungsgut (4) in einem ersten Transportschritt (T1) über dem Behälter (3) positioniert wird, wobei in einem Dekontaminationsschritt (A) das Behandlungsgut (4) in den Behälter (3) abgesenkt wird, so dass das Behandlungsgut (4) vollständig von dem in dem Behälter (3) befindlichen Reaktivgas (R) bedeckt ist, und für eine definierte Dekontaminationszeit in dem Behälter (3) belassen wird, so dass das Behandlungsgut (4) mittels des Reaktivgases (R) dekontaminiert wird und nach Ablauf der Dekontaminierungszeit dem Behälter (3) entnommen wird wobei in einem zweiten Transportschritt (T2) das Behandlungsgut (4) über der Spülposition (6) positioniert wird, wobei das Behandlungsgut (4) anschließend in einem Spülschritt (B) auf die Spülposition (6) abgesenkt wird, und mit dem Spülgas gespült wird, wobei dem Behandlungsgut (4) anhaftende Reste von Reaktivgas (R) entfernt werden, und der Spülposition (6) entnommen wird, wobei in einem dritten Transportschritt (T3) das Behandlungsgut (4) zur Entnahme weitertransportiert wird.

4. Verfahren nach einem der vorangegangenen Ansprüche, wobei das Plasma zur Erzeugung des Reaktivgases (R) aus dem Prozessgas mittels Mikrowellen gezündet wird.

5. Verfahren nach Anspruch 1, **dadurch gekennzeichnet, dass** das Spülgas sterile Luft ist.

## Claims

1. Immersion decontamination method for the bio-decontamination of objects (4) by means of a reactive gas (R), comprising the following steps:
• Generating a reactive gas (R) from a process gas by igniting a plasma, wherein ambient air is used as the process gas and the reactive gas (R) comprises nitrogen dioxide (N02), and wherein the reactive gas (R) has a higher density than air at the same pressure and temperature,
• Filling a container (3) with the reactive gas (R) so that the container (3) is almost completely or at least partially filled with the reactive gas (R),
• Bio-decontamination of the objects to be treated (4) in the container (3) by means of the reactive gas (R), wherein the objects to be treated (4) are lowered into the container (3) filled with reactive gas (R) so that the objects to be treated are completely covered by the reactive gas, wherein, after a decontamination period has elapsed, the objects to be treated are removed from the container,
• wherein, after the decontamination time has elapsed, the objects are flushed with a flushing gas at a flushing position in order to remove the reactive gas, so that residues of the reactive gas are removed from the objects to be treated.

2. Method according to claim 1, wherein the container (3) is connected via a reactive gas line to a plasma source (2) for generating a plasma, and wherein reactive gas (R) generated by the plasma source (2) is introduced into the container (3) via the reactive gas line.

3. Method according to one of the preceding claims, wherein the objects to be treated (4) are positioned above the container (3) in a first transport step (T1), wherein in a decontamination step (A) the objects to be treated (4) are lowered into the container (3) so that the objects to be treated (4) are completely covered by the reactive gas (R) located in the container (3) and is left in the container (3) for a defined decontamination time, so that the objects to be treated (4) are decontaminated by means of the reactive gas (R) and, after the decontamination time has elapsed, are removed from the container (3), wherein in a second transport step (T2) the objects to be treated (4) are positioned above the flushing position (6), wherein the objects to be treated (4) are then lowered to the flushing position (6) in a flushing step (B) and flushed with the flushing gas, wherein residues of reactive gas (R) adhering to the objects to be treated (4) are removed, and are removed from the flushing position (6), wherein in a third transport step (T3) the objects to be treated (4) are transported further for removal.

4. Method according to one of the preceding claims, wherein the plasma for generating the reactive gas (R) is ignited from the process gas by means of microwaves.

5. Method according to claim 1, **characterized in that** the flushing gas is sterile air.

## Revendications

1. Procédé de décontamination par immersion pour la décontamination biologique de matériau devant être traité (4) au moyen d'un gaz réactif (R), comprenant les étapes de :
• génération d'un gaz réactif (R) à partir d'un gaz de processus par allumage d'un plasma, dans lequel de l'air ambiant est utilisé en tant que gaz de processus et le gaz réactif (R) présente du dioxyde d'azote (NO₂), et dans lequel
le gaz réactif (R) présente une densité supérieure à l'air à pression identique et température identique,
• remplissage d'un récipient (3) du gaz réactif (R) de sorte que le récipient (3) est presque entièrement ou au moins partiellement rempli du gaz réactif (R),
• décontamination biologique du matériau devant être traité (4) dans le récipient (3) au moyen du gaz réactif (R), dans lequel le matériau devant être traité (4) est abaissé dans le récipient (3) rempli du gaz réactif (R) de sorte que le matériau devant être traité est entièrement recouvert du gaz réactif, dans lequel après écoulement d'une durée de décontamination, le matériau devant être traité est prélevé du récipient,
• dans lequel le matériau devant être traité est rincé avec un gaz de rinçage à une position de rinçage après écoulement de la durée de décontamination pour l'élimination du gaz réactif pour éliminer des restes du gaz réactif du matériau devant être traité.

2. Procédé selon la revendication 1, dans lequel le récipient (3) est connecté à une source de plasma (2) par le biais d'une conduite de gaz réactif pour la génération d'un plasma et dans lequel du gaz réactif (R) généré au moyen de la source de plasma (2) est introduit dans le récipient (3) par le biais de la conduite de gaz réactif.

3. Procédé selon une des revendications précédentes, dans lequel le matériau devant être traité (4) est positionné au-dessus du récipient (3) dans une première étape de transport (T1), dans lequel le matériau devant être traité (4) est abaissé dans le récipient (3) dans une étape de décontamination (A) de sorte que le matériau devant être traité (4) est entièrement recouvert du gaz réactif (R) se trouvant dans le récipient (3), et est laissé dans le récipient (3) pour une durée de décontamination définie de sorte que le matériau devant être traité (4) est décontaminé au moyen du gaz réactif (R) et est prélevé du récipient (3) après écoulement de la durée de décontamination, dans lequel le matériau devant être traité (4) est positionné au-dessus de la position de rinçage (6) dans une deuxième étape de transport (T2), dans lequel le matériau devant être traité (4) est ensuite abaissé sur la position de rinçage (6) dans une étape de rinçage (B) et est rincé avec le gaz de rinçage, dans lequel des restes de gaz réactif (R) adhérant au matériau devant être traité (4) sont éliminés, et est prélevé de la position de rinçage (6), dans lequel le matériau devant être traité (4) continue d'être transporté pour le prélèvement dans une troisième étape de transport (T3).

4. Procédé selon une des revendications précédentes, dans lequel le plasma pour la génération du gaz réactif (R) à partir du gaz de processus est allumé au moyen de micro-ondes.

5. Procédé selon la revendication 1, **caractérisé en ce que** le gaz de rinçage est de l'air stérile.
